# EUROPEAN PATENT APPLICATION

(11) **EP 4 817 568 A1**
(43) Date of publication of application: **07.10.2026**
(21) Application number: 24896195.5
(22) Date of filing: 04.11.2024
(51) Int. Cl.: A61F 2/90

(54) **SHEATHED STENT AND DELIVERY SYSTEM**

(30) Priority: 30.11.2023 CN 202311636008
(71) Applicant: Lifetech Scientific (Shenzhen) Co., Ltd., Shenzhen, Guangdong 518063 (CN)
(72) Inventor: CHEN, Erchong, Shenzhen, Guangdong 518063 (CN); SHAO, Ge, Shenzhen, Guangdong 518063 (CN)
(74) Representative: Michalski Hüttermann & Partner mbB
(86) International application number: PCT/CN2024/129771
(87) International publication number: WO 2025/113104

(57) **Abstract**

The present application provides a covered stent (100) and a delivery system (1000). The covered stent (100) includes a covered body (10), a first wave ring (20), and a balancing structure (50). The covered body (10) includes an end section (11) having an open end (111). The first wave ring (20) is connected to the end section (11). The first wave ring (20) includes a first waveform section (21) and a second waveform section (22). The radial support strength of the first waveform section (21) is less than the radial support strength of the second waveform section (22). A region where the end section (11) on the covered stent (100) is located is a first region (100a). The balancing structure (50) is provided in the first region (100a) and configured to increase the radial support strength of a region in the first region (100a) that is coaxial with the first waveform section (21). The covered stent (100) and the delivery system (1000) provided in the present application allow a more uniform deployment of the first region (100a) and the covered stent (100) in a circumferential direction, thus reducing the irritation and damage to blood vessels during deployment.

## Description

### Technical Field

The present application relates to the technical field of medical devices, and in particular, to a covered stent and a delivery system.

### Background Art

Aortic aneurysm and aortic dissection are currently life-threatening diseases. If not actively treated, the aortic aneurysm and dissection will continue to enlarge and eventually rupture, leading to severe complications and death. With the increasing number of patients suffering from hypertension, hyperlipidemia, and hyperglycemia, the incidence of aortic aneurysm and aortic dissection is significantly rising.

Traditional open surgery for treating aortic aneurysm and aortic dissection involves large trauma, high mortality, long operation time, high postoperative complication rate, and high surgical difficulty. In contrast, endovascular therapy offers advantages such as less trauma, fewer postoperative complications, shorter operation time, and lower surgical difficulty, and has gradually become the primary method for treating aortic aneurysm and aortic dissection. Endovascular therapy involves implanting a covered stent into the aorta through a delivery device, isolating the vascular lesion outside the covered stent, and directing blood flow through the interior of the covered stent, thereby protecting the blood vessel.

However, an end section of the covered stent in the related art is provided with a first wave ring. The first wave ring includes a first waveform section and a second waveform section connected to the first waveform section, and the radial support strength of the first waveform section is less than the radial support strength of the second waveform section. When the covered stent is deployed from the delivery device, the first wave ring may rebound under its own elastic force. During the rebound process, the first wave ring may drive the end section of the covered stent to contact the inner wall of the blood vessel. Since the radial support strength of the first waveform section is less than the radial support strength of the second waveform section, the second waveform section rebounds faster than the first waveform section, so that the end section of the covered stent is deployed unevenly in the circumferential direction, resulting in non-uniform forces exerted by the end section of the covered stent on the inner wall of the blood vessel in the circumferential direction. This may lead to concentrated stress within the blood vessel, easily causing impact or damage to the blood vessel during deployment, resulting in patient discomfort or even vasospasm.

### Summary of the Invention

In view of the shortcomings in the foregoing technology, the present application provides a covered stent and a delivery system.

An embodiment of the present application provides a covered stent, including:
a covered body including an end section having an open end;
a first wave ring connected to the end section and including a first waveform section and a second waveform section, the radial support strength of the first waveform section being less than the radial support strength of the second waveform section; a region where the end section of the covered stent is located being a first region; and
a balancing structure provided in the first region and configured to increase the radial support strength of a region in the first region that is coaxial with the first waveform section.

In the covered stent according to the embodiment of the present application, the balancing structure includes a second wave ring connected to the end section.

In the covered stent according to the embodiment of the present application, an axial region of the second wave ring at least partially overlaps an axial region of the first wave ring.

In the covered stent according to the embodiment of the present application, the second wave ring includes a third wave and a fourth wave; the radial support strength of the third wave is less than the radial support strength of the fourth wave; the fourth wave is axially aligned with the first waveform section, and the third wave is axially aligned with the second waveform section.

In the covered stent according to the embodiment of the present application, the first waveform section includes a first wave, and the second waveform section includes a second wave; the wave height of the first wave is greater than the wave height of the second wave, and the wave angle of the first wave is equal to the wave angle of the second wave; the wave height of the third wave is equal to the wave height of the fourth wave, and the wave angle of the third wave is greater than the wave angle of the fourth wave.

In the covered stent according to the embodiment of the present application, the second wave ring is configured to form a closed structure, and the closed structure is axially aligned with the first waveform section.

In the covered stent according to the embodiment of the present application, the balancing structure further includes a third wave ring connected to the end section, and the third wave ring intersects and cooperates with the second wave ring to form the closed structure.

In the covered stent according to the embodiment of the present application, the wire diameter of the third wave ring is less than the wire diameter of the second wave ring.

In the covered stent according to the embodiment of the present application, in the first region, there is at least one first intersection point in the region that is coaxial with the first waveform section, where the first intersection point is formed by intersection between the first waveform section and the balancing structure or by the balancing structure itself.

In the covered stent according to the embodiment of the present application, in the first region, there are a plurality of second intersection points in the region that are coaxial with the second waveform section, and the number of first intersection points is greater than the number of second intersection points, where the second intersection point is formed by intersection between the second waveform section and the balancing structure or by the balancing structure itself.

In the covered stent according to the embodiment of the present application, the balancing structure is at least partially provided on an end face of the covered body or adjacent to the end face of the covered body for supporting the end face of the covered body.

An embodiment of the present application further provides a delivery system, including:
a delivery device; and
the covered stent according to any one of the foregoing aspects, the delivery device being configured to deliver the covered stent.

According to the covered stent and the delivery system provided in the embodiment of the present application, the covered stent includes the balancing structure connected to the end section, and the balancing structure is configured to increase the radial support strength of the region in the first region that is coaxial with the first waveform section. Therefore, the balancing structure can balance the radial support strength of the region in the first region that is coaxial with the first waveform section and the radial support strength of the region in the first region that is coaxial with the second waveform section, and reduce the difference in deployment speed between the region in the first region that is coaxial with the first waveform section and the region in the first region that is coaxial with the second waveform section, thereby enabling more uniform deployment of the first region and the covered stent in the circumferential direction, resulting in more uniform forces exerted by the first region and the covered stent on the inner wall of the blood vessel in the circumferential direction, reducing concentrated stress on the inner wall of the blood vessel, and minimizing irritation and damage to the blood vessel during deployment.

It should be understood that the foregoing general description and the following detailed description are merely exemplary and explanatory, and do not limit the disclosed content of the embodiments of the present application.

### Brief Description of the Drawings

To more clearly describe the technical solutions in embodiments of the present application, the drawings required in the descriptions of the embodiments will be briefly introduced below. Obviously, the drawings described below are some embodiments of the present application, and a person skilled in the art may obtain other drawings according to these drawings without involving any creative efforts.
FIG. 1 is a schematic structural diagram of a delivery system according to an embodiment of the present application;
FIG. 2 is a schematic structural diagram of a covered stent according to an embodiment of the present application;
FIG. 3 is a schematic diagram of a partial structure of a covered stent according to an embodiment of the present application;
FIG. 4 is a schematic diagram of a partial structure of a covered stent according to an embodiment of the present application;
FIG. 5 is a schematic diagram of a partial structure of a covered stent according to an embodiment of the present application;
FIG. 6 is a schematic diagram of a partial structure of a covered stent according to an embodiment of the present application;
FIG. 7 is a schematic diagram of a partial structure of a covered stent according to an embodiment of the present application;
FIG. 8 is a schematic diagram of a partial structure of a covered stent according to an embodiment of the present application;
FIG. 9 is a schematic diagram of a partial structure of a covered stent according to an embodiment of the present application;
FIG. 10 is a schematic diagram of a position of a connecting portion according to an embodiment of the present application;
FIG. 11 is a schematic diagram of a partial structure of a covered stent according to an embodiment of the present application;
FIG. 12 is a schematic diagram of a position of a connecting portion according to an embodiment of the present application;
FIG. 13 is a schematic diagram of a position of a connecting portion according to an embodiment of the present application and an enlarged view of part A therein.
FIG. 14 is a schematic diagram of a partial structure of a covered stent according to an embodiment of the present application;
FIG. 15 is a schematic diagram of a partial structure of a covered stent according to an embodiment of the present application;
FIG. 16 is a schematic diagram of a partial structure of an expanded end section according to an embodiment of the present application;
FIG. 17 is a schematic diagram of a partial structure of an expanded end section according to an embodiment of the present application;
FIG. 18 is a schematic diagram of a partial structure of an expanded end section according to an embodiment of the present application;
FIG. 19 is a schematic diagram of a partial structure of an expanded end section according to an embodiment of the present application;
FIG. 20 is a schematic diagram of a partial structure of an expanded end section according to an embodiment of the present application;
FIG. 21 is a schematic diagram of a partial structure of an expanded end section according to an embodiment of the present application; and
FIG. 22 is a schematic diagram of a partial structure of an expanded end section according to an embodiment of the present application.

Description of reference numerals:
1000-delivery system;
100-covered stent; 100a-first region;
10-covered body; 11-end section; 111-open end; 1111-proximal open end; 1112-distal open end; 112-end face; 1121-first partial end face; 1122-second partial end face; 11a-convex region; 12-main body section;
20-first wave ring; 21-first waveform section; 21a-first wave; 211-first wave rod; 212-second wave rod; 213-first wave peak; 22-second waveform section; 22a-second wave; 221-third wave rod; 222-fourth wave rod; 223-second wave peak; 2011-part of first wave ring 20 at least partially exposed outside covered body 10 in axial direction;
30-support frame;
40-connecting portion; 41-connecting line; 42-connecting band; 43-connecting hole; 44-connecting slit; 441-open end of connecting slit; 45-connecting membrane; 450-insertion opening; 451-suture;
50-balancing structure; 51-second wave ring; 511-third wave; 5111-fifth wave rod; 5112-sixth wave rod; 5113-third wave peak; 512-fourth wave; 5121-seventh wave rod; 5122-eighth wave rod; 5123-fourth wave peak; 52-third wave ring; 53-closed structure; 54-fourth wave ring;
61-first intersection point; 62-second intersection point; and
200-delivery device; 201-sheath core; 202-sheath; and 203-anchoring piece.

### Detailed Description of the Invention

The technical solutions in embodiments of the present application will be clearly and completely described below with reference to the accompanying drawings in the embodiments of the present application. Obviously, the described embodiments are some rather than all of the embodiments of the present application. All other examples obtained by a person skilled in the art based on the embodiments of the present application without making creative efforts fall within the protection scope of the present application.

In the description of the present application, it is to be understood that orientation or positional relationships indicated by the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", and the like is based on the orientation or positional relationships shown in the drawings, and are intended only to facilitate and simplify the description of the present invention, and are not intended to indicate or imply that the apparatus or element referred to must have a particular orientation, constructed and operated in a particular orientation, and therefore are not to be construed as limitations of the present application. In addition, the terms "first" and "second" are used for descriptive purposes only and are not to be construed as indicating or implying relative importance or implicitly indicating the number of technical features indicated. Thus, a feature defined as "first" or "second" may explicitly or implicitly include one or more such features. In the description of the present application, the meaning of "a plurality of" means two or more, unless otherwise explicitly and specifically limited.

It should also be understood that the terms used in the specification of the present application are merely intended to describe specific embodiments, but are not intended to limit the present application. As used in the specification of the present application and the appended claims, unless the context clearly indicates otherwise, the singular forms "a", "an", and "the" are intended to include the plural forms.

It is further understood that the term "and/or" as used in the specification of the present application and the appended claims refers to any combination and all possible combinations of one or more of the associated listed items, and includes such combinations.

Hereinafter, some implementations of the present application will be described in detail with reference to the drawings. The following embodiments and features in the embodiments may be combined with each other without conflict.

For ease of description, the terms "proximal end" and "distal end" are defined herein as conventional terms used in the field of interventional medicine. Specifically, the "distal end" indicates an end where blood flows out, and the "proximal end" indicates an end where blood flows in. For example, after the covered stent is implanted into a lumen, blood flows from the proximal end of the covered stent toward the distal end. An "axial direction" indicates its longitudinal direction, or a direction in which an interventional device is advanced and retracted. A "radial direction" indicates a direction perpendicular to the "axial direction".

The lumen is illustrated by taking a blood vessel as an example. The blood vessel may include, but is not limited to, at least one of the following: ascending aorta, aortic arch, descending aorta, thoracic aorta, abdominal aorta, vein, and the like. A person skilled in the art will recognize that the use of blood vessels is for illustrative purposes only and is not intended to limit the present application. The solutions of the present application are applicable to various human lumens or animal lumens. For example, the human lumens may include digestive tract lumens or blood vessels. Various improvements and variations based on the teachings of the present application fall within the protection scope of the present application.

The "wave ring" in the embodiment of the present application is a closed or non-closed wave-shaped annular structure and may also be referred to as a wave-shaped annular structure. It may be provided on an inner wall and/or an outer wall of a covered body of a covered stent, and the wave ring and the covered body are connected through at least one of the following connection methods: suturing, bonding, hot melting, or the like. Illustratively, at least part of the wave ring may be made of a material having good tensile and resilient properties and good biocompatibility. For example, the material includes at least one of the following: known materials used in implanted medical devices, various biocompatible materials, such as alloys of two or more single metals selected from cobalt, chromium, nickel, titanium, magnesium, and iron, stainless steel, or nickel-titanium-tantalum alloys, or other biocompatible elastic materials. The wave ring has a radial expansion capability and may achieve radial contraction under external force. After removal of the external force, it may return to and maintain its initial shape through self-expansion or mechanical expansion (e.g., expansion through balloon dilation), thereby enabling the device to closely fit the inner wall of the lumen through its radial support force after implantation into the lumen. The waveform of the wave ring is not limited, and includes, for example, at least one of the following: Z-shaped waves, M-shaped waves, V-shaped waves, and sine waves. The wave ring includes a plurality of wave peaks (also referred to as proximal vertices), a plurality of wave troughs (also referred to as distal vertices), and wave rods connecting adjacent wave peaks and wave troughs. One vertex (proximal vertex or distal vertex) and two wave rods connected to the vertex form one wave.

It will be understood that the "number of waves" referred to in the embodiments of the present application refers to the number of wave peaks or the number of wave troughs. The "wave height" refers to a vertical distance between a wave peak and an adjacent lowest wave trough. The "wave angle" refers to an included angle between two adjacent wave rods connected to the same vertex.

It will be understood that the radial support force (in N) may be measured using a radial support force tester, such as the RX550-100 model radial support force tester manufactured by Machine Solution Inc (MSI). Taking a first wave ring as an example, the first wave ring is placed in a radial crimping device of the tester. During the test, the radial crimping device continuously and uniformly compresses the first wave ring in the radial direction until the first wave ring is compressed to 20% of its original diameter, and the value of the radial support force of the first wave ring in this case is measured.

The radial support force of a first wave of the first wave ring may be measured to be less than the radial support force of a second wave of the first wave ring through the following method. A first test wave ring including a plurality of first waves is fabricated, with the diameter of the first test wave ring being the same as the diameter of the first wave ring. A second test wave ring including a plurality of second waves is fabricated, with the diameter of the second test wave ring being the same as the diameter of the first wave ring. The radial support force of the first test wave ring and the radial support force of the second test wave ring are separately measured to measure that the radial support force of the first test wave ring is less than the radial support force of the second test wave ring. Since both the first wave and the second wave are located on the first wave ring, and the maximum axial length of the first wave ring is a fixed value, it may be calculated that the radial support force of the first wave of the first wave ring is less than the radial support force of the second wave of the first wave ring.

The radial support strength (in Pa) mentioned in the embodiments of the present application may be calculated using the following formula: Radial support strength = Radial support force ÷ Maximum axial length at measured position. It will be understood that when a plurality of wave rings are provided in the axial direction at the measured position, the closer the spacing between the plurality of wave rings in the axial direction, the greater the radial support strength.

It should be noted that A being equal to B refers to A being equal to B within the range of assembly and/or installation errors, or A being substantially equal to B.

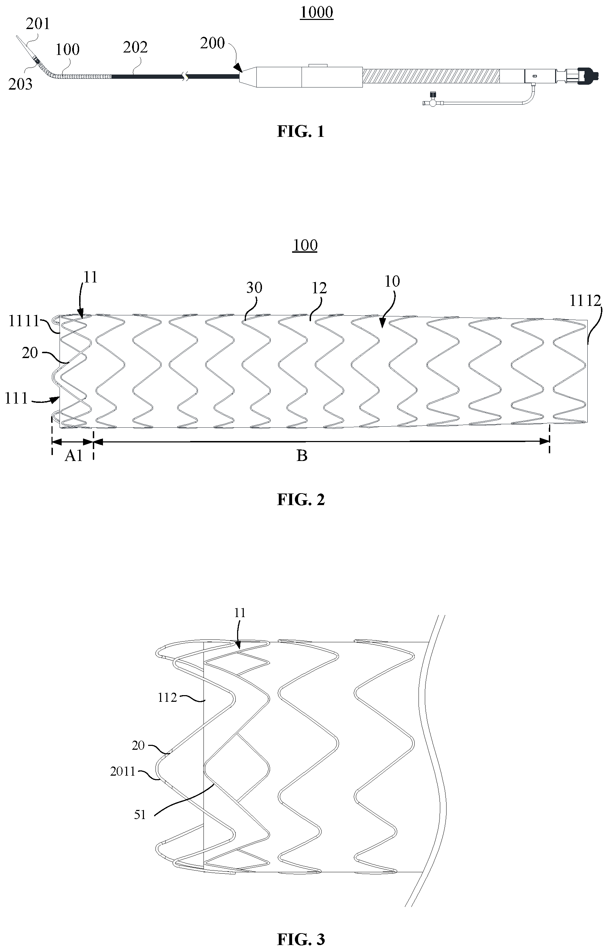

Referring to FIG. 1, an embodiment of the present application provides a delivery system 1000, including a covered stent 100 and a delivery device 200. The delivery device 200 is configured to deliver the covered stent 100 to a vascular lesion position, thereby isolating the vascular lesion outside the covered stent 100, thus protecting the blood vessel.

Referring to FIG. 1, in some embodiments, the delivery device 200 includes a sheath core 201 and a sheath 202. The sheath core 201 extends through the sheath 202, and the sheath 202 can accommodate and deliver the covered stent 100. When the covered stent 100 needs to be delivered to the vascular lesion position, the covered stent 100 may first be connected to an anchoring piece 203 of the delivery device 200, and then compressed and loaded between the sheath 202 and the sheath core 201 to facilitate delivery of the covered stent 100.

It will be understood that the covered stent 100 can be radially compressed and loaded into the sheath 202 of the delivery device 200. After the covered stent 100 is implanted at the lesion position through the delivery device 200, the covered stent 100 can isolate the blood flow from the lesion position and eliminate the influence of blood pressure on the lesion position, thereby achieving a therapeutic effect.

Referring to FIG. 2, in some embodiments, the covered stent 100 includes a covered body 10 and a first wave ring 20. The covered body 10 includes an end section 11 having an open end 111. The first wave ring 20 is connected to and relatively fixed to the end section 11. It will be understood that the covered body 10 includes a hollow tubular structure, and the hollow cavity of the covered body 10 forms a channel for the blood flow.

Referring to FIG. 2, in some implementations, the covered body 10 has at least one proximal open end 1111 and one distal open end 1112. According to actual requirements, the first wave ring 20 may be provided at the end section 11 where the proximal open end 1111 of the covered body 10 is located, may be provided at the end section 11 where the distal open end 1112 is located, or may be simultaneously provided at end sections 11 at two ends of the covered body 10. Illustratively, the first wave ring 20 is provided at the proximal open end 1111 of the covered body 10.

Referring to FIG. 2, in some embodiments, the covered body 10 further includes a main body section 12, and the covered stent 100 further includes a support frame 30. The end section 11 is connected to the main body section 12. The support frame 30 is connected to the main body section 12 and configured to support the main body section 12. The end section 11 is located at the proximal end of the main body section 12. Alternatively, the end section 11 is located at the distal end of the main body section 12. The support frame 30 has a radial expansion capability and may achieve radial contraction under external force. After removal of the external force, it may return to and maintain its initial shape through self-expansion, thereby enabling the support frame to closely fit the inner wall of the blood vessel through its radial support force after implantation into the blood vessel. Illustratively, the main body section 12 includes a tubular main body covering, and the support frame 30 includes at least one support wave ring.

Illustratively, the main body covering and the end covering may each have a single-layer structure or a multi-layer structure, and are not limited herein. The main body covering and the end covering may each be made of at least one of the following materials: polytetra fluoroethylene (PTFE), polyethylene terephthalate (PET), or other polymer materials with good biocompatibility. The main body covering may be fixed to an inner surface and/or outer surface of the support frame 30 by suturing, bonding, hot melting, or the like, to reconstruct fluid channel, isolate lesion regions of the blood vessel, and the like.

Illustratively, an axial region of the end section 11 is shown as A1 in FIG. 2, and an axial region of the main body section 12 is shown as B in FIG. 2.

In some implementations, the end section 11 includes a tubular end covering, and the open end 111 of the end section 11 may be enclosed by an edge of the end covering or an end face 112 (see FIG. 3). Illustratively, the end section 11 is provided with an end support assembly, and the end covering may be fixed to an inner surface and/or outer surface of the end support assembly by suturing, bonding, hot melting, or the like. The end support assembly includes the first wave ring 20 connected to the end section 11. In other implementations, the end support assembly further includes other wave rings connected to the end section 11, to further increase the anchoring force of the covered body 10 in the radial direction and further improve the stability of the covered stent 100 during use.

In some implementations, the end section 11 is switchable between a first state and a second state. The radial dimension of the end section 11 in the first state is less than that in the second state. The second state may be a natural expansion state, that is, a natural extension state without the action of external human force. Alternatively, it may be a state in which the end section is radially compressed to a certain extent but not yet completely expanded. In either case, the radial dimension of the end section in the second state is greater than that in the first state.

Referring to FIG. 3, in some implementations, at least part of the first wave ring 20 is exposed outside the end section 11 in the axial direction. When the end section 11 is in the first state, at least part of the first wave ring 20 extends beyond the end face 112 of the end section 11 in the axial direction, so that the first wave ring 20 is at least partially exposed outside the covered body 10 in the axial direction. The connection between the first wave ring 20 exposed outside the covered body 10 and the delivery device 200 is convenient, so that the covered stent 100 can be loaded at the delivery device 200 simply, easily, and quickly. In this embodiment, a part 2011 of the first wave ring 20 at least partially exposed outside the covered body 10 in the axial direction is inclined toward a central axis of the covered body 10, so that the part 2011 of the first wave ring 20 at least partially exposed outside the covered body 10 in the axial direction is easier to be deployed from the delivery device 200.

Referring to FIG. 3, illustratively, the first wave ring 20 may partially extend beyond the covered body 10 in the axial direction, and the part of the first wave ring 20 beyond the covered body 10 may be bent toward the axial direction of the covered stent 100, so that the part of the first wave ring 20 beyond the covered body 10 does not contact the inner wall of the blood vessel after the covered stent 100 is implanted into the blood vessel, thereby preventing the first wave ring 20 from causing damage or great irritation to the inner wall of the blood vessel.

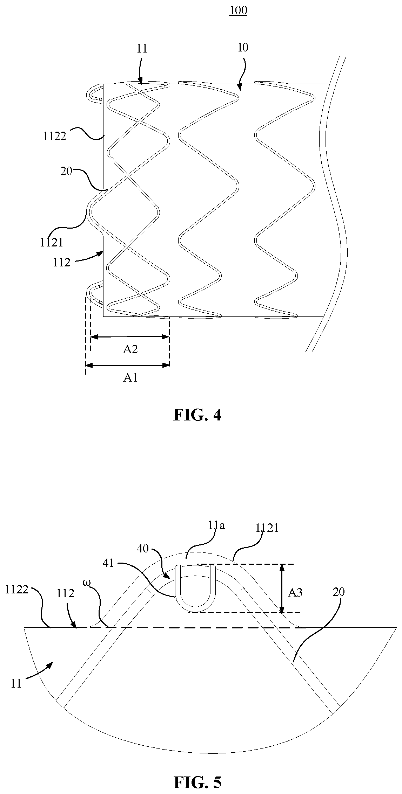

Referring to FIGS. 4 and 5, in some embodiments, the first wave ring 20 is located within the end section 11. The covered stent 100 further includes a connecting portion 40. The connecting portion 40 is formed on at least one of the end section 11 and the first wave ring 20 and is configured to be connected to the delivery device 200. An end face 112 of the end section 11 includes first partial end faces 1121 and second partial end faces 1122 which are connected to each other. The first partial end face 1121 and the second partial end face 1122 are provided in a circumferential direction of the covered body 10. Compared with the second partial end face 1122, the first partial end face 1121 protrudes in a direction away from the main body section 12, that is, protrudes outwardly. A convex region 11a is formed between a surface where the second partial end face 1122 is located and the first partial end face 1121. At least one end of the connecting portion 40 away from the main body section 12 is provided in the convex region 11a for driving the convex region 11a to be positioned on the delivery device 200. In this case, the surface where the second partial end face 1122 is located is a plane.

In this embodiment, since the first wave ring 20 is connected to and relatively fixed to the end section 11, the first wave ring 20 and the end section 11 form an integral part. The connecting portion 40 is formed on at least one of the end section 11 and the first wave ring 20, that is, the connecting portion 40 is connected to the integral part formed by the end section 11 and the first wave ring 20. In this case, the connecting portion 40 is connected to the delivery device 200, and the delivery device 200 is connected to the integral part formed by the end section 11 and the first wave ring 20 through the connecting portion 40, which may buffer the force exerted by the delivery device 200 that may cause relative displacement between the first wave ring 20 and the end section 11, thus maintaining a fixed relative position between the first wave ring 20 and the end section 11. Therefore, the end section 11 and the first wave ring 20 of the covered stent 100 maintain their shapes after deployment of the covered stent 100 so that the covered stent 100 can better fit against the inner wall of the blood vessel, ensuring wall apposition of the covered stent 100, thereby improving the sealing efficacy of the covered stent 100, preventing endoleaks, and reducing surgical risk.

Meanwhile, on the basis of ensuring the wall apposition, the covered stent 100 of this embodiment can be compressed in a loaded state and fixedly connected to the anchoring piece 203 of the delivery device 200 through the connecting portion 40. The connecting portion 40 is connected to the anchoring piece 203, and at least the end of the connecting portion 40 away from the main body section 12 is provided in the convex region 11a, thereby driving the convex region 11a to be positioned on the anchoring piece 203, forming a relatively tight contact. Compared with a planar end face 112 of the covered stent 100, the convex region 11a is a region axially enclosed by the surface where the second partial end face 1122 is located and the first partial end face 1121. Other parts of the covered stent 100 may be at least partially offset relative to the connecting portion 40 or the convex region 11a in the axial direction of the covered stent 100, resulting in a small radial compression diameter of the part of the covered stent 100 connected to the anchoring piece 203. This facilitates loading the covered stent 100 into a sheath 202 with a small diameter, thus facilitating delivery of the covered stent 100 to the lesion position by the delivery device 200. In addition, the number of parts of the covered stent 100 connected to the anchoring piece 203 is small, so that unnecessary interference between the covered stent 100 and the anchoring piece 203 of the delivery device 200 generated during assembly and/or deployment may be reduced, the assembly and/or deployment efficiency is improved, and the damage to the covered stent 100 and/or the delivery device 200 is reduced.

Referring to FIG. 4, in some embodiments, the first wave ring 20 is located within the end section 11. In this case, the axial region A2 of the first wave ring 20 is located within the axial region A1 of the end section 11, that is, the end section 11 can completely cover the first wave ring 20. There is no bare stent portion of the first wave ring 20 exposed outside the covered body 10 in the axial direction, thereby preventing the problem caused by partial exposure of the first wave ring 20 in the axial direction of the covered stent 100, such that the exposed part of the first wave ring 20 is easily pulled by the delivery device 200 and then undergoes incomplete reversible deformation when the covered stent 100 is compressed. This ensures that the first wave ring 20 can restore and maintain its shape after deployment, thereby ensuring that the end section 11 and the covered stent 100 can maintain their shapes after deployment, ensuring good wall apposition of the covered stent 100, improving the sealing efficacy of the covered stent 100, and preventing endoleaks. For example, at least a part of wave peaks of the first wave ring 20 is flush with the end face 112 of the end section 11. Alternatively, the wave peaks of the first wave ring 20 are provided adjacent to the end face 112 of the end section 11, and the axial region A2 of the first wave ring 20 is located within the axial region A1 of the end section 11, so that the open end 111 of the end section 11 better maintains its shape and can effectively fit against the inner wall of the blood vessel.

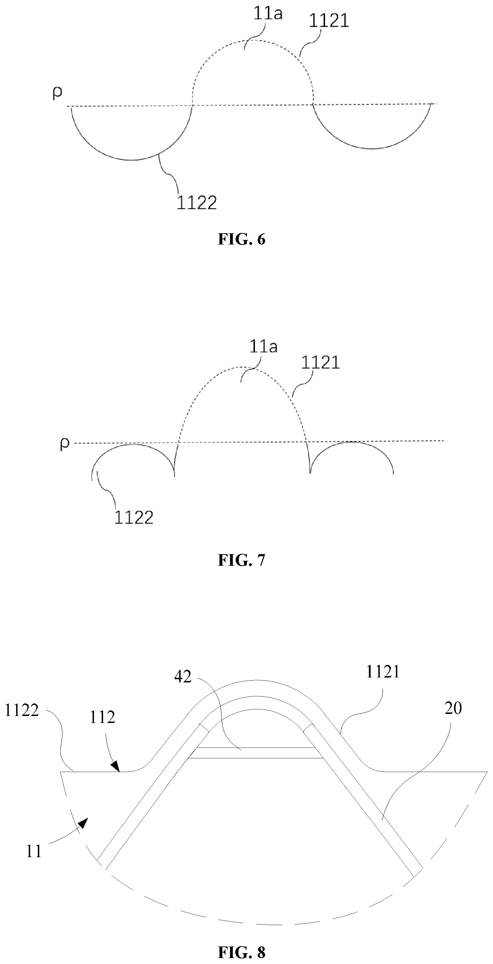

In some implementations, the surface where the second partial end face 1122 is located is a plane. Alternatively, the second partial end face 1122 is a concave curved surface or a convex curved surface. Illustratively, if the second partial end face 1122 is a plane, the surface where the second partial end face 1122 is located is shown as ω in FIG. 5, and the surface ω where the second partial end face 1122 is located and the first partial end face 1121 (indicated by a dashed line) enclose to form the convex region 11a. Referring to FIG. 6, if the second partial end face 1122 is concave relative to the first partial end face 1121, that is, the second partial end face 1122 is a concave curved surface, a surface ρ where a cross section of the concave curved surface farthest away from the main body section 12 in a direction perpendicular to the axial direction of the covered stent is located and the first partial end face 1121 (indicated by a dashed line in FIG. 6) enclose to form the convex region 11a. Referring to FIG. 7, if the second partial end face 1122 is a convex curved surface, a surface ρ where a cross section of the convex curved surface farthest away from the main body section 12 in the direction perpendicular to the axial direction of the covered stent is located and the first partial end face 1121 (indicated by a dashed line in FIG. 7) enclose to form the convex region 11a.

In some embodiments, the number of first partial end faces 1121 and second partial end faces 1122 may be designed according to actual requirements, such as one, two, three, or more. Referring to FIG. 4, in some implementations, there are at least two first partial end faces 1121, and a plurality of first partial end faces 1121 are provided at intervals in the circumferential direction of the covered stent 100. Each first partial end face 1121 is correspondingly provided with at least one connecting portion 40, to improve the connection reliability between the covered stent 100 and the delivery device 200. It will be understood that a second partial end face 1122 is connected between two adjacent first partial end faces 1121. Illustratively, the plurality of first partial end faces 1121 are provided at equal intervals in the circumferential direction, so that the radial support performance of the end section 11 is more uniform, thereby ensuring good wall apposition of the covered stent 100.

It will be understood that the connecting portion 40 is formed on at least one of the end section 11 and the first wave ring 20, including the following cases: the connecting portion 40 is formed on the end section 11 or the first wave ring 20; and the connecting portion 40 is formed on the end section 11 and the first wave ring 20.

In some embodiments, the connecting portion 40 is configured to hook onto the anchoring piece 203 of the delivery device 200, and the assembly and deployment of the connecting portion 40 and the anchoring piece 203 are simple and rapid. Illustratively, one of the anchoring piece 203 and the connecting portion 40 may include a hook, and the other includes a hollow structure or a hole structure that mates with the hook.

Referring to FIG. 5 or FIG. 8, in some embodiments, the connecting portion 40 includes a connecting line 41 or a connecting band 42, and the connecting line 41 or the connecting band 42 is connected to at least one of the end section 11 and the first wave ring 20. For the connecting portion 40 with this structure, the force applied by the delivery device 200 at the connecting portion 40 can be relatively uniformly distributed to the integral part formed by the first wave ring 20 and the end section 11, thereby ensuring that the end section 11 and the covered stent 100 can maintain their shapes after deployment, allowing the covered stent 100 to better fit against the inner wall of the blood vessel, and ensuring the wall apposition of the covered stent 100. Illustratively, the connecting line 41 or the connecting band 42 is connected to at least the first wave ring 20, so that the force applied by the delivery device 200 at the connecting portion 40 can be relatively uniformly distributed to connection positions between the first wave ring 20 and the end section 11, thereby ensuring that the end section 11 of the covered stent 100 and the covered stent 100 can maintain their shapes after deployment, and improving the wall apposition of the covered stent 100. For example, the connecting line 41 is provided at the wave peak of the first wave ring 20, and on the basis of ensuring good wall apposition of the covered stent 100, the space occupied by the first wave ring 20 and the first partial end face 1121 can be fully utilized, so that the covered stent 100 has a compact structure and occupies a small space. The connecting line 41 or the connecting band 42 may be provided on an inner wall of the end section 11, or may be provided on an outer wall of the end section 11. Illustratively, the connecting line 41 or the connecting band 42 is provided on the inner wall of the end section 11. When the delivery device 200 implants the covered stent 100 into the blood vessel, the injury caused by the connecting portion 40 to the inner wall of the blood vessel can be reduced, thereby avoiding significant irritation or damage caused by the connecting portion 40 to the inner wall of the blood vessel. Materials of the connecting line 41 or the connecting band 42 include, but are not limited to, metal materials, polymer materials, or metal and polymer composite materials.

In this embodiment, the connecting line 41 or the connecting band 42 is located within the convex region 11a. With the connecting line 41 or the connecting band 42 located within the convex region 11a, after the connecting line 41 or the connecting band 42 is hooked onto the anchoring piece 203, the convex region 11a can be more precisely positioned onto the anchoring piece 203, reducing or avoiding contact between non-convex regions of the covered stent 100 and the anchoring piece 203, thereby allowing selection of a sheath with a smaller inner diameter.

Illustratively, the connecting line 41 may include any suitable filament-like or wire-like structure. The connecting band 42 may include a strip-shaped or belt-shaped structure, for example, including a strip-shaped covering. One end of the connecting band 42 is connected to the first wave ring 20 and/or the end section 11, the other end of the connecting band 42 is connected to the first wave ring 20 and/or the end section 11, and a middle portion of the connecting band 42 is configured to be hooked by the anchoring piece 203 of the delivery device 200.

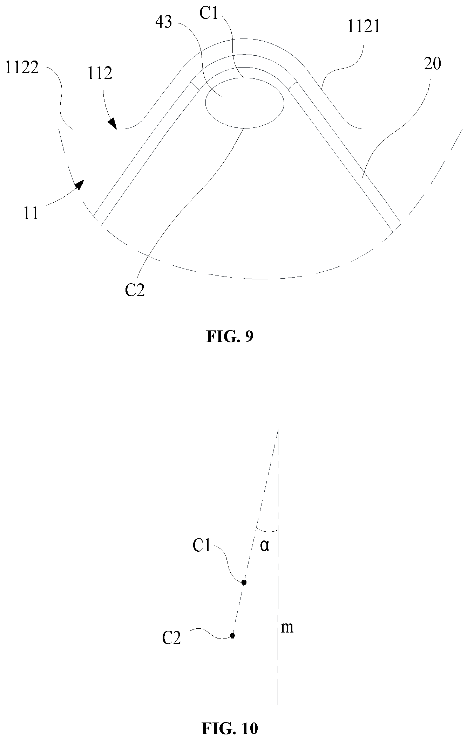

Referring to FIG. 9, in some embodiments, the connecting portion 40 includes a connecting hole 43. The anchoring piece 203 of the delivery device 200 passes through the connecting hole 43 to fix the covered stent 100. Illustratively, the connecting hole 43 extends through the end section 11. In this embodiment, the shape of the connecting hole 43 may be circular, square, or irregular.

It will be understood that at least one end of the connecting hole 43 away from the main body section 12 is arranged in the convex region 11a, including the following cases. Case 1, the connecting hole 43 is arranged in the convex region 11a. Case 2, the end of the connecting hole 43 away from the main body section 12 is arranged in the convex region 11a, while the end of the connecting hole 43 close to the main body section 12 is arranged in another region in the end section 11 except the convex region 11a.

Since the anchoring piece 203 is hooked onto the end of the connecting hole 43 away from the main body section 12, the end of the connecting hole 43 close to the main body section 12 may be arranged anywhere in the end section 11.

Referring to FIGS. 2, 9, and 10, in some embodiments, the connecting portion 40 includes a first end C1 away from the main body section 12 and a second end C2 close to the main body section 12. The first end C1 is closer to an axis m of the covered stent 100 than the second end C2, so that the connecting portion 40 has a certain inclination angle, enabling easier deployment and disengagement of the anchoring piece 203 of the delivery device 200 from the connecting portion 40. In other embodiments, the distances from the first end C1 and the second end C2 of the connecting portion 40 to the axis m of the covered stent 100 may be equal. Alternatively, the first end C1 of the connecting portion 40 may be farther from the axis m of the covered stent 100 than the second end C2.

Referring to FIGS. 9 and 10, illustratively, the connecting portion 40 includes the connecting hole 43. A wall of the connecting hole 43 has a first end C1 (i.e., a proximal end of the wall of the connecting hole 43) and a second end C2 (i.e., a distal end of the wall of the connecting hole 43). An included angle α between a straight line connecting the first end C1 and the second end C2 of the connecting hole 43 and the axis m of the covered stent 100 is an acute angle, and the first end C1 of the wall of the connecting hole 43 is closer to the axis m of the covered stent 100 than the second end C2 of the wall of the connecting hole 43, so that the connecting hole 43 has a certain inclination angle, enabling easier deployment and disengagement of the anchoring piece 203 of the delivery device 200 from the connecting hole 43. In other embodiments, the included angle α between the straight line connecting the first end C1 and the second end C2 of the connecting hole 43 and the axis m of the covered stent 100 may not be an acute angle. For example, the straight line connecting the first end C1 and the second end C2 of the connecting hole 43 may be parallel to the axis m of the covered stent 100.

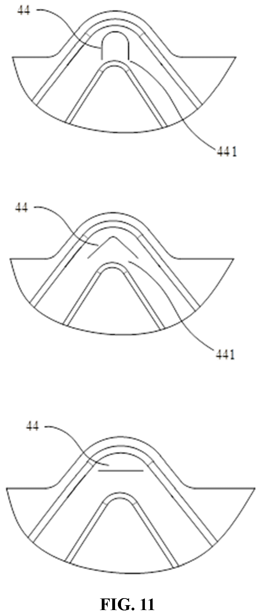

In FIG. 11, illustratively, the connecting portion 40 includes a connecting slit 44, and the connecting slit 44 is formed in the end section 11. Specifically, the connecting slit 44 is cut in the end section 11, and extends through the end section 11. The anchoring piece 203 of the delivery device 200 passes through the connecting slit 44 to fix the covered stent 100. In this embodiment, the shape of the connecting slit 44 may be linear, U-shaped, V-shaped, W-shaped, or the like. The U-shaped, V-shaped, and W-shaped slits are connecting slits 44 having an open end 441. The open end 441 of the connecting slit 44 is close to the main body section 12, thereby facilitating hooking of the anchoring piece 203 of the delivery device 200 from one end of the connecting slit 44 away from the main body section 12. It will be understood that the connecting slit 44 having the open end 441 may be opened to form a larger opening. Thus, compared with the linear-shaped connecting slit 44, the connecting slit 44 having the open end 441 makes it easier for the anchoring piece 203 of the delivery device 200 to extend into the connecting slit 44 for hooking.

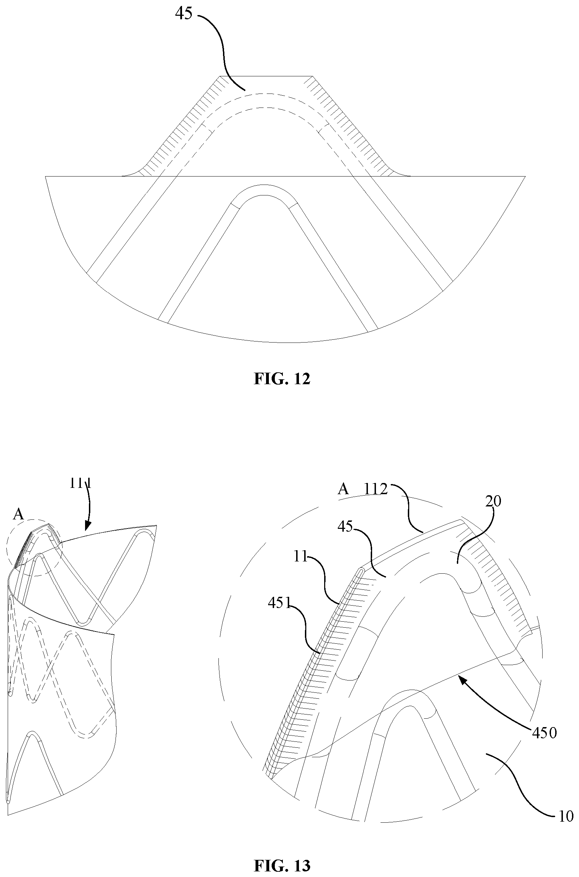

With reference to FIGS. 12 and 13, illustratively, the connecting portion includes a connecting membrane 45, and part of the first wave ring 20 is provided between the end section 11 and the connecting membrane 45. An insertion opening 450 is arranged between the end section 11 and the connecting membrane 45 for receiving the anchoring piece 203 of the delivery device 200 to achieve connection. In this embodiment, the connecting membrane 45 and the end section 11 are integrally formed, that is, the connecting membrane 45 extends from the covered body 10. Specifically, the covered body 10 extends toward the open end 111, folds inward at the end surface 112, and then extends away from the open end 111 to form the connecting membrane 45. The connecting membrane 45 and two sides of the end section 11 are sutured and fixed through a suture 451, and the insertion opening 450 is formed at the position of the connecting membrane 45 away from the open end 111. In another embodiment, the connecting membrane 45 and the end section 11 are not integrally formed. The connecting membrane 45 is a separate membrane and may be connected to at least one of the end section 11 or the first wave ring 20 by suturing or adhesive bonding. In one embodiment, the connecting membrane 45 may be made of a material having a radiopaque function, and may be configured to indicate the position of the open end 111 of the covered stent 100. Specifically, a radiopaque element may be connected to the connecting membrane 45, or the connecting membrane 45 may be immersed in a radiopaque solution.

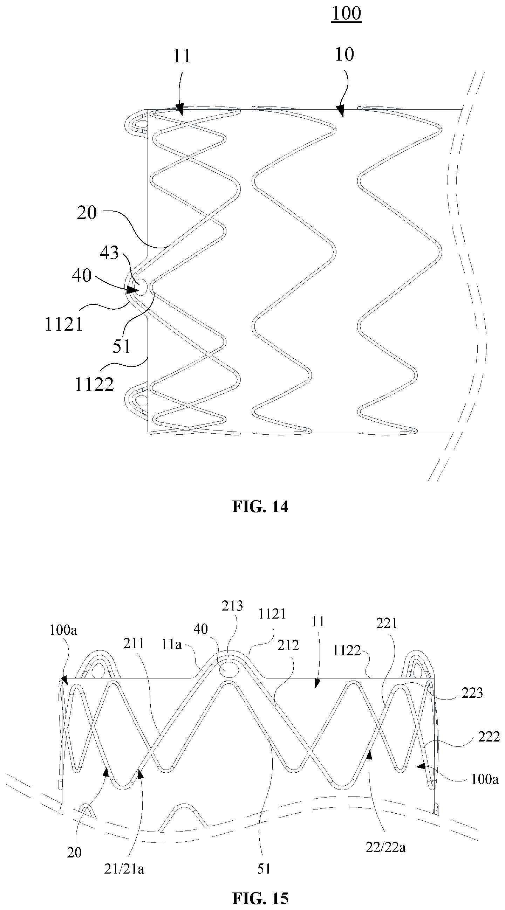

Referring to FIG. 14, in some embodiments, the covered stent 100 further includes a second wave ring 51. The second wave ring 51 is connected to the end section 11. The connecting portion 40 includes a connecting hole 43, and one wave peak of the second wave ring 51 is provided adjacent to the connecting hole 43. Thus, during assembly and deployment of the covered stent 100, the second wave ring 51 can better support the covered body 10 around the connecting hole 43, avoiding the situation that the connecting portion 40 or the end section 11 cannot be disengaged from the anchoring piece 203 of the delivery device 200 due to the deformation of the connecting hole 43. It will be understood that one wave peak of the first wave ring 20 may also be provided adjacent to the connecting slit 44.

Referring to FIG. 14, in some embodiments, one wave peak of the first wave ring 20 is provided adjacent to the connecting hole 43, and in the axial direction of the covered stent 100, the connecting hole 43 is arranged between one wave peak of the first wave ring 20 and one wave peak of the second wave ring 51. Thus, during assembly and deployment of the covered stent 100, both the first wave ring 20 and the second wave ring 51 can effectively support the covered body 10 around the connecting hole 43, thereby effectively avoiding the situation that the connecting portion 40 or the end section 11 cannot be disengaged from the anchoring piece 203 of the delivery device 200 due to the deformation of the connecting hole 43. Illustratively, one wave peak of the first wave ring 20, the connecting hole 43, and one wave peak of the second wave ring 51 are sequentially arranged in the axial direction of the covered stent 100, and one wave peak of the first wave ring 20 is closer to the end face 112 of the end section 11 than one wave peak of the second wave ring 51. In other embodiments, the second wave ring 51 may intersect or not intersect with the first wave ring 20, and the wave peak of the second wave ring 51 has a certain circumferential distance from the connecting hole 43.

In some embodiments, the first wave ring 20 includes a wave-shaped annular structure formed by a first support wire. The first wave ring 20 is elastically deformable and may be partially or entirely made of an elastic material. For example, the first wave ring 20 may be made of a material having good tensile and resilient properties and good biocompatibility, such as nickel-titanium, stainless steel, and the like. Due to good tensile and resilient properties, the first wave ring 20 inherently possesses an elastic force that allows it to recover from deformation The first wave ring 20 has an elastically deformable property, thereby increasing the anchoring force in the radial direction of the covered body 10, and improving the stability of the covered stent 100 during use. In other embodiments, the first wave ring 20 may also be made of a non-elastic material. For example, adjacent wave rods in the first wave ring 20 made of the non-elastic material are rotatably connected by providing a pivot piece. In this case, the first wave ring 20 itself does not have an elastic force, and its deformation is driven by other wave rings of the covered body 10 and/or the covered stent 100 having self-expansion capability.

Referring to FIG. 15, in some embodiments, the first wave ring 20 includes a first waveform section 21. In some implementations, the first waveform section 21 includes at least one first wave 21a. Illustratively, the first waveform section 21 includes first wave rods 211 and second wave rods 212, and adjacent first wave rod 211 and second wave rod 212 are connected to the same first wave peak 213 to form a first wave 21a.

Illustratively, the position of the first waveform section 21 or the first wave 21a is axially aligned with the position of the first partial end face 1121 so that the first waveform section 21 or the first wave 21a can support the first partial end face 1121.

Referring to FIG. 15, in some implementations, the first wave ring 20 further includes a second waveform section 22. Illustratively, the first waveform section 21 is connected to the second waveform section 22. In some implementations, the second waveform section 22 includes at least one second wave 22a. Illustratively, the first wave ring 20 includes third wave rods 221 and fourth wave rods 222, and adjacent third wave rod 221 and fourth wave rod 222 are connected to the same second wave peak 223 to form a second wave 22a.

Illustratively, the position of the second waveform section 22 or the second wave 22a is axially aligned with the position of the second partial end face 1122 so that the second waveform section 22 or the second wave 22a can support the second partial end face 1122.

Illustratively, the first partial end face 1121, the wave peak of the first wave 21a, and at least a part of the connecting portion 40 are sequentially arranged in the axial direction of the covered stent 100, resulting in a compact structure of the covered stent 100 and good wall apposition of the end section 11. In other implementations, the first wave ring 20 may also intersect with the end face 112 of the end section 11.

Referring to FIG. 15, in some embodiments, the wave peak of the first wave 21a is located within the convex region 11a so that the first wave 21a can effectively support the convex region 11a. Illustratively, the first wave 21a is located within the end section 11 to avoid the first wave 21a being at least partially exposed outside the end section 11. In addition, the first wave 21a is connected to the end section 11 and relatively fixed to the end section 11 so that the first wave 21a is always engaged with the end section 11, thereby avoiding the problem that the first wave 21a is easily pulled by the delivery device 200 and then undergoes incomplete reversible deformation when the covered stent 100 is compressed. This ensures that the first wave 21a and the end section 11 can restore and maintain their shapes after deployment, thereby ensuring good wall apposition of the covered stent 100, improving the sealing efficacy of the covered stent 100, and preventing endoleaks. Compared with the first wave 21a being at least partially exposed outside the end section 11 in the axial direction, in this embodiment, the axial region of the first wave 21a is located within the axial region of the end section 11 so that collision or friction between the first wave ring 20 and the delivery device 200 may be reduced, thereby reducing unnecessary interference between the covered stent 100 and the delivery device 200 during assembly and/or deployment. For example, the wave peak of the first wave 21a is adjacent to a convex vertex of the first partial end face 1121, or the wave peak of the first wave 21a is located at the first partial end face 1121, to effectively support the convex region 11a of the covered body 10 and the end section of the covered stent 100, so that the covered stent 100 has better wall apposition, and the risk of endoleaks is reduced after implantation of the covered stent 100 into the blood vessel.

Illustratively, the second wave 22a is located within the end section 11, thereby preventing the problem caused by the second wave 22a being at least partially exposed outside the end section 11, such that the exposed part of the second wave 22a is easily pulled by the delivery device 200 and then undergoes incomplete reversible deformation when the covered stent 100 is compressed. This ensures that the second wave 22a can restore and maintain its shape after deployment, thereby ensuring that the end section 11 and the covered stent 100 can maintain their shapes after deployment, ensuring good wall apposition of the covered stent 100, improving the sealing efficacy of the covered stent 100, and preventing endoleaks. For example, the wave peak of the second wave 22a is provided adjacent to the second partial end face 1122, or the wave peak of the second wave 22a is located at the second partial end face 1122, so that the second wave 22a can effectively support the second partial end face 1122 of the covered body 10 and the end section of the covered stent 100. Thus, the covered stent 100 has better wall apposition, and the risk of endoleaks is reduced after implantation of the covered stent 100 into the blood vessel.

It will be understood that the wire diameter, wave height, number of waves, and wave angle of the first wave ring 20 may be set according to actual requirements. For example, the wire diameter of the first wave ring 20 ranges from 0.3 mm to 0.45 mm, such as 0.3 mm, 0.4 mm, 0.45 mm, or any other suitable value between 0.3 mm and 0.45 mm. The wave height of the first wave 21a and/or the second wave 22a ranges from 1 mm to 15 mm, such as 1 mm, 5 mm, 15 mm, or any other suitable value between 1 mm and 15 mm. The number of first waves 21a and/or second waves 22a ranges from 3 to 30, such as 3, 6, 10, 20, 30, or any other suitable value between 3 and 30. The greater the number of waves of the wave ring, the greater the radial support force on the end portion of the covered stent 100. Thus, the wall apposition effect of the end portion of the covered stent 100 may be effectively improved.

Referring to FIG. 15, in some embodiments, the first wave ring 20 includes a first waveform section 21 and a second waveform section 22. The radial support strength of the first waveform section 21 is less than the radial support strength of the second waveform section 22. A region where the end section 11 of the covered stent 100 is located is a first region 100a. It will be understood that the first region 100a includes the end section 11 and wave rings provided on the end section 11.

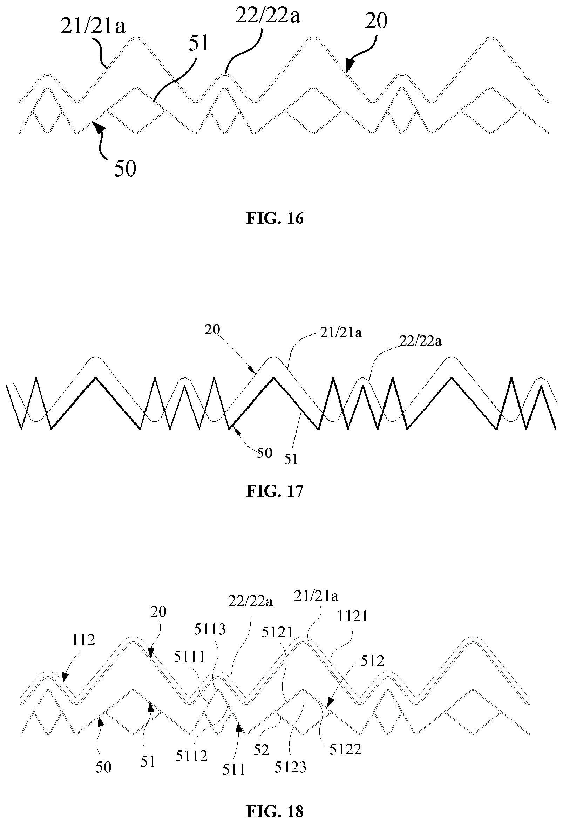

It will be understood that after the covered stent 100 is deployed from the delivery device 200, the first wave ring 20 will rebound under its own elastic force, and during the rebound process, the first wave ring 20 will drive the first region 100a to contact the inner wall of the blood vessel. Since the radial support strength of the first waveform section 21 is less than the radial support strength of the second waveform section 22, the second waveform section 22 rebounds faster than the first waveform section 21, so that the first region 100a is deployed unevenly in the circumferential direction, resulting in non-uniform forces exerted by the first region 100a on the inner wall of the blood vessel in the circumferential direction. This may lead to concentrated stress within the blood vessel, easily causing impact or damage to the blood vessel during deployment, resulting in patient discomfort or even vasospasm. In addition, the non-uniform deployment of the first region 100a in the circumferential direction also affects the shape of the covered stent 100 after compression and deployment, leading to insufficient wall apposition of the first region 100a, increasing the risk of endoleaks, and increasing the surgical risk. To this end, referring to FIG. 16, in some embodiments, the covered stent 100 further includes a balancing structure 50. The balancing structure 50 is provided in the first region 100a and configured to increase the radial support strength of a region in the first region 100a that is coaxial with the first waveform section 21. In this embodiment, the balancing structure 50 is located on the surface of the end section 11 or between the inner and outer surfaces of the end section 11.

According to the covered stent 100 in the foregoing embodiment, the covered stent 100 includes the balancing structure 50 connected to the end section 11, and the balancing structure 50 is configured to increase the radial support strength of the region in the first region 100a that is coaxial with the first waveform section 21. Therefore, the balancing structure 50 can balance the radial support strength of the region in the first region 100a that is coaxial with the first waveform section 21 and the radial support strength of the region in the first region 100a that is coaxial with the second waveform section 22, and reduce the difference in deployment speed between the region in the first region 100a that is coaxial with the first waveform section 21 and the region in the first region 100a that is coaxial with the second waveform section 22, thereby enabling more uniform deployment of the first region 100a and the covered stent 100 in the circumferential direction, resulting in more uniform forces exerted by the first region 100a and the covered stent 100 on the inner wall of the blood vessel in the circumferential direction, reducing concentrated stress on the inner wall of the blood vessel, and minimizing irritation or damage to the blood vessel during deployment. In addition, uniform deployment of the first region 100a in the circumferential direction enables better maintenance of the shape of the end section 11 or the first region 100a after compression and deployment of the covered stent 100, ensuring good wall apposition of the first region 100a and the covered stent 100, preventing endoleaks, and reducing the surgical risk.

In some implementations, the radial support strength of the first wave 21a is less than the radial support strength of the second wave 22a. Illustratively, the wave height of the first wave 21a is greater than the wave height of the second wave 22a, and the wave angle of the first wave 21a is equal to the wave angle of the second wave 22a, so that the radial support strength of the first wave 21a is less than the radial support strength of the second wave 22a. Illustratively, when the axial region of the first wave ring 20 is located within the axial region of the end section 11, the wave height of the first wave 21a is greater than the wave height of the second wave 22a, and the wave angle of the first wave 21a is equal to the wave angle of the second wave 22a, so that the first wave 21a can better support the first partial end face 1121 and the radial support strength of the first wave 21a is less than the radial support strength of the second wave 22a. When the first wave ring 20 is at least partially exposed outside the axial region of the end section 11 in the axial direction, the wave height of the first wave 21a is greater than the wave height of the second wave 22a, and the wave angle of the first wave 21a is equal to the wave angle of the second wave 22a, so that the first wave 21a can be more easily and conveniently connected to the anchoring piece 203 of the delivery device 200 and the radial support strength of the first wave 21a is less than the radial support strength of the second wave 22a. In other implementations, the wave height of the first wave 21a may also be less than or equal to the wave height of the second wave 22a. The wave angle of the first wave 21a may also be greater than or less than the wave angle of the second wave 22a.

In other embodiments, the radial support strength of the first waveform section 21 is greater than or equal to the radial support strength of the second waveform section 22. The radial support strength of the first wave 21a is greater than or equal to the radial support strength of the second wave 22a.

Referring to FIGS. 16 and 17, in some embodiments, the balancing structure 50 includes a second wave ring 51. The second wave ring 51 is connected to the end section 11 and configured to increase the radial support strength of the region in the first region 100a that is coaxial with the first waveform section 21, thereby balancing the radial support strength of the region in the first region 100a that is coaxial with the first waveform section 21 and the radial support strength of the region in the first region 100a that is coaxial with the second waveform section 22, and reducing the difference in deployment speed between the region in the first region 100a that is coaxial with the first waveform section 21 and the region in the first region 100a that is coaxial with the second waveform section 22, and thus enabling more uniform deployment of the first region 100a and the covered stent 100 in the circumferential direction. In addition, the second wave ring 51 can further serve to reinforce the support of the end section 11, ensuring good wall apposition of the first region 100a after deployment, and preventing endoleaks caused by a bird-beak shape of the first region 100a. In other embodiments, the balancing structure 50 is not necessarily a wave ring structure, but may be a separate element, such as a mesh or block made of metal or plastic.

In some implementations, the balancing structure 50 is located within the end section 11 so that the balancing structure 50 can effectively increase the radial support strength of the region in the first region 100a that is coaxial with the first waveform section 21. Illustratively, the first wave ring 20 and the balancing structure 50 are provided at intervals in the axial direction of the covered stent 100 to avoid the problem that the balancing structure 50 interferes with the connection and deployment of the first wave ring 20 and the delivery device 200.

In some implementations, the second wave ring 51 may also intersect with the first wave ring 20, as shown in FIG. 15; and/or, the wave peak of the second wave ring 51 may also intersect with the end face 112 of the open end 111, as shown in FIG. 3; and/or, the second wave ring 51 is located within the end section 11. In some implementations, referring to FIGS. 15 and 17, the proximal end of the second wave ring 51 or the proximal end of the balancing structure 50 may be provided adjacent to the end face 112 of the end section 11, specifically adjacent to the second partial end face 1122, so that the second wave ring 51 or at least a part of the balancing structure 50 can support the end face 112 of the end section 11 to a certain extent. Thus, the end face 112 of the end section 11 can maintain a good shape after compression and deployment of the covered stent 100, resulting in better wall apposition of the covered stent 100, and reducing the risk of endoleaks.

Referring to FIG. 18, in some implementations, at least two of the end face 112 of the end section 11, the first wave ring 20, and the second wave ring 51 are provided at intervals in the axial direction. Illustratively, the end face 112 of the end section 11, the first wave ring 20, and the second wave ring 51 are sequentially provided at intervals in the axial direction of the covered stent 100, thus facilitating manufacturing of the covered stent 100.

In some implementations, the axial region of the second wave ring 51 at least partially overlaps with the axial region of the first wave ring 20, so that the axial distance between the second wave ring 51 and the first wave ring 20 is reduced, and the radial support strength at the proximal end of the covered stent 100 is increased, thereby enhancing the radial support force of the first region 100a, enhancing the anchoring property of the proximal end of the covered stent 100 in the blood vessel, minimizing displacement of the proximal end of the covered stent 100, improving the sealing property of the covered stent 100, and resulting in a more compact structure of the covered stent 100.

Referring to FIG. 18, in some embodiments, the second wave ring 51 is connected to the end section 11 and located within the end section 11. For example, at least a part of wave peaks of the second wave ring 51 is flush with the end face 112 of the end section 11. Alternatively, the wave peak of the second wave ring 51 is provided at a certain distance from the end face 112 of the end section 11, and the end face 112 of the end section 11 is provided at the proximal side relative to the wave peak of the second wave ring 51. Illustratively, as shown in the figure, the second wave ring 51 is located within the end section 11, and the wave peak of the first waveform section 21 and one wave peak of the second wave ring 51 are provided at intervals in the axial direction of the covered stent 100.

Referring to FIG. 18, in some embodiments, the second wave ring 51 includes third waves 511 and fourth waves 512. The radial support strength of the third wave 511 is less than the radial support strength of the fourth wave 512, the fourth wave 512 is axially aligned with the first waveform section 21, and the third wave 511 is axially aligned with the second waveform section 22, so that the second wave ring 51 can serve to balance the circumferential support strength of the first region 100a. Thus, the first region 100a is more uniformly deployed in the circumferential direction, thereby achieving more uniform forces exerted by the first region 100a and the covered stent 100 on the inner wall of the blood vessel in the circumferential direction.

Illustratively, at least one third wave 511 may be provided between two adjacent fourth waves 512, and at least one fourth wave 512 may be provided between two adjacent third waves 511.

In some embodiments, the wave height of the third wave 511 is equal to the wave height of the fourth wave 512, and the wave angle of the third wave 511 is less than the wave angle of the fourth wave 512, so that the radial support strength of the third wave 511 is less than the radial support strength of the fourth wave 512. In other embodiments, the wave height of the third wave 511 may also be less than or greater than the wave height of the fourth wave 512, and the wave angle of the third wave 511 may also be greater than or equal to the wave angle of the fourth wave 512. This is not limited herein, as long as the radial support strength of the third wave 511 is less than the radial support strength of the fourth wave 512.

Illustratively, the first waveform section 21 includes the first wave 21a, and the second waveform section 22 includes the second wave 22a. The wave height of the first wave 21a is greater than the wave height of the second wave 22a, and the wave angle of the first wave 21a is equal to the wave angle of the second wave 22a. The wave height of the third wave 511 is equal to the wave height of the fourth wave 512, and the wave angle of the third wave 511 is less than the wave angle of the fourth wave 512, so that the second wave ring 51 can effectively balance the radial support strength of the region in the first region 100a that is coaxial with the first waveform section 21 and the radial support strength of the region in the first region 100a that is coaxial with the second waveform section 22, and reduce the difference in deployment speed between the region in the first region 100a that is coaxial with the first waveform section 21 and the region in the first region 100a that is coaxial with the second waveform section 22, thus enabling more uniform deployment of the first region 100a in the circumferential direction. In addition, both the first wave ring 20 and the second wave ring 51 can serve to reinforce the support of the end section 11, effectively ensuring good wall apposition of the first region 100a after deployment.

Referring to FIG. 18, illustratively, the third wave 511 includes a fifth wave rod 5111 and a sixth wave rod 5112, and adjacent fifth wave rod 5111 and sixth wave rod 5112 are connected to the same third wave peak 5113 to form the third wave 511. The fourth wave 512 includes a seventh wave rod 5121 and an eighth wave rod 5122, and adjacent seventh wave rod 5121 and eighth wave rod 5122 are connected to the same fourth wave peak 5123 to form the fourth wave 512.

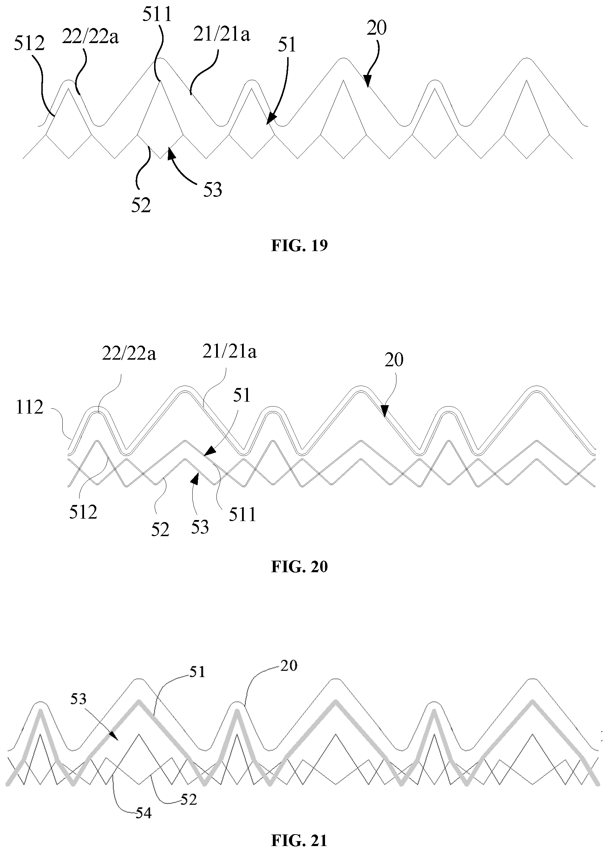

Illustratively, the wave rod of the third wave 511 includes a straight line, a polyline, or a curve. For example, referring to FIG. 18, the wave rod of the third wave 511 and/or the wave rod of the fourth wave 512 includes a straight line. As another example, referring to FIG. 19, the wave rod of the third wave 511 and/or the wave rod of the fourth wave 512 includes a polyline. The third wave 511 with a curved wave rod can extend more easily without interfering with other waves, thereby more easily supporting the end face 112 of the end section 11 to a certain extent, and further enabling the end face 112 of the end section 11 to maintain a good shape after compression and deployment of the covered stent 100.

Illustratively, the number of wave peaks of the first wave ring 20 and the number of wave peaks of the second wave ring 51 may be the same or different, which is not limited herein. The number of first waves 21a and the number of third waves 511 may be the same or different. The number of second waves 22a and the number of fourth waves 512 may be the same or different.

Referring to FIG. 19, in some embodiments, the balancing structure 50 further includes a third wave ring 52. The third wave ring 52 is connected to the end section 11 and configured to increase the radial support strength of the region in the first region 100a that is coaxial with the first waveform section 21, thereby balancing the radial support strength of the region in the first region 100a that is coaxial with the first waveform section 21 and the radial support strength of the region in the first region 100a that is coaxial with the second waveform section 22, and reducing the difference in deployment speed between the region in the first region 100a that is coaxial with the first waveform section 21 and the region in the first region 100a that is coaxial with the second waveform section 22, and thus enabling more uniform deployment of the first region 100a and the covered stent 100 in the circumferential direction. In addition, the third wave ring 52 can further serve to reinforce the support of the end section 11, ensuring good wall apposition of the first region 100a after deployment, and preventing endoleaks caused by a bird-beak shape of the first region 100a. In this embodiment, the third wave ring 52 is also an annular wave ring, and the third wave ring 52 and the second wave ring 51 have a partially overlapping region.

Referring to FIG. 19, in some embodiments, the second wave ring 51 is configured to form a closed structure 53, and the closed structure 53 is axially aligned with the first waveform section 21. It will be understood that the second wave ring 51 and the third wave ring 52 are both fixed to the covered body 10, and the closed structure 53 is also fixed to the covered body 10, especially the vertices of the closed structure 53 are fixed. Therefore, when the covered stent 100 is radially compressed, a proximal vertex of the closed structure 53 moves toward the proximal direction, and a distal vertex of the closed structure 53 moves toward the distal direction. In this case, a covered part enclosed by the closed structure 53 is simultaneously pulled axially toward the proximal end and axially toward the distal end. When the covered part within the closed structure 53 is pulled to its limit, further radial compression of the closed structure 53 becomes difficult. Therefore, the covered part enclosed by the closed structure 53 resists radial compression of the closed structure 53, making it difficult for the closed structure 53 to be compressed radially. As a result, the radial support strength of the closed structure 53 is greater than the radial support strength of a non-closed structure.

Referring to FIG. 19, in some embodiments, the third wave ring 52 intersects and cooperates with the second wave ring 51 to form the closed structure 53, thereby further increasing the radial support strength of the region in the first region 100a that is coaxial with the first waveform section 21, and making the deployment of the first region 100a as uniform as possible in the circumferential direction.

Referring to FIG. 19, in some implementations, one wave trough of the third wave ring 52 and one wave peak of the second wave ring 51 are provided at intervals in the axial direction of the covered stent 100, and the third wave ring 52 intersects with the second wave ring 51 so that the second wave ring 51 cooperates with the third wave ring 52 to form the closed structure 53.

Referring to FIG. 19, in some embodiments, the axial region of the third wave ring 52 and the axial region of the second wave ring 51 at least partially overlap to improve the support performance of the balancing structure 50, so that the balancing structure 50 can better balance the radial support strength of the first region 100a and the covered stent 100 in the circumferential direction, and further improve the deployment uniformity of the first region 100a and the covered stent 100 in the circumferential direction.

Illustratively, the heights of the waves of the third wave ring 52 may be the same, different, or partially the same, and are not limited herein.

Referring to FIG. 19, in some embodiments, the wave height of the wave of the third wave ring 52 is less than that of the second wave ring 51, and the axial region of the third wave ring 52 is located within the axial region of the second wave ring 51. Thus, the balancing structure 50 can not only balance the radial support strength of the covered stent 100 in the circumferential direction, but also facilitate a more compact structure of the covered stent 100. In addition, a reduced axial distance between the third wave ring 52 and the second wave ring 51 may increase the radial support strength at the proximal end of the covered stent 100, thereby enhancing the anchoring property of the proximal end of the covered stent 100 in the blood vessel, and minimizing displacement of the covered stent 100 in the blood vessel. Illustratively, a distal end (at least a part of wave troughs) of the third wave ring 52 is flush with a distal end (at least a part of wave troughs) of the second wave ring 51.

In some implementations, the number of wave peaks of the third wave ring 52 is greater than or equal to the number of wave peaks of the second wave ring 51 to improve the radial support performance of the balancing structure 50, so that the balancing structure 50 can better balance the radial support strength of the first region 100a and the covered stent 100 in the circumferential direction. For example, the number of wave peaks of the third wave ring 52 is equal to twice the number of wave peaks of the second wave ring 51. In other implementations, the number of wave peaks of the third wave ring 52 may also be less than the number of wave peaks of the second wave ring 51.

Illustratively, the wave peak of the third wave ring 52 is farther away from the end face 112 of the end section 11 than the wave peak of the second wave ring 51, so that the connection of the second wave ring 51 and the third wave ring 52 to the end section 11 is convenient, simple, and easy.

It will be understood that at least a part of the wave peaks of the third wave ring 52 may be provided at any suitable position. For example, at least a part of the wave peaks of the third wave ring 52 is located on at least one of the fifth wave rod 5111, the sixth wave rod 5112, the seventh wave rod 5121, and the eighth wave rod 5122. As another example, the wave peak of the third wave ring 52 may be provided at at least one of the following positions: between the fifth wave rod 5111 and the sixth wave rod 5112; between the sixth wave rod 5112 and the seventh wave rod 5121; between the seventh wave rod 5121 and the eighth wave rod 5122; and between the eighth wave rod 5122 and the fifth wave rod 5111.

Illustratively, the axial region of the third wave ring 52 is located within the axial region of the end section 11. The axial region of the third wave ring 52 is located within the axial region of the second wave ring 51 to better increase the radial support strength of the region in the first region 100a that is coaxial with the first waveform section 21, so that the force exerted by the first region 100a on the inner wall of the blood vessel in the circumferential direction is more uniform, alleviating or avoiding concentrated stress on the inner wall of the blood vessel, and thus alleviating or avoiding damage to the blood vessel. In addition, the end section 11 may be deployed more uniformly, ensuring that the end section 11 can maintain its shape well after deployment, enabling the end section 11 to better fit against the inner wall of the blood vessel, improving the wall apposition of the end section 11 of the covered stent 100, and thereby preventing endoleaks.

The shape of the closed structure 53 may be set according to actual requirements, such as including at least one of the following: a quadrangle, a pentagon, a hexagon, other polygons, and other irregular closed shapes. For example, referring to FIG. 19, the shape of the closed structure 53 includes a quadrangle or rhombus. As another example, referring to FIG. 20, the shape of the closed structure 53 includes a hexagon.

In one embodiment, the balancing structure 50 includes at least one of the following: the second wave ring 51, the third wave ring 52, the fourth wave ring 54, and other wave rings. Referring to FIG. 21, illustratively, the balancing structure 50 includes a second wave ring 51, a third wave ring 52, and a fourth wave ring 54. The second wave ring 51, the third wave ring 52, and the fourth wave ring 54 are each connected to the end section 11, and the fourth wave ring 54 may intersect with the second wave ring 51 and/or the third wave ring 52. The balancing structure 50 including a plurality of wave rings can effectively increase the radial support strength of the region in the first region 100a that is coaxial with the first waveform section 21. When the radial support strength between circumferentially adjacent waveform sections of the first wave ring 20 differs significantly, or when the overall radial support strength of the proximal end of the covered stent 100 is insufficient, a balancing structure 50 including a plurality of wave rings may be provided at a corresponding position to make the overall radial support strength of the proximal end (or the first region 100a) of the covered stent 100 as appropriate as possible and enable more uniform deployment of the proximal end of the covered stent 100 in the circumferential direction. In addition, the wave rings of the balancing structure 50 can also independently support the end section 11, ensuring good wall apposition of the first region 100a after deployment, and preventing endoleaks caused by a bird-beak shape of the end of the covered stent 100. Illustratively, the second wave ring 51, the third wave ring 52, and the fourth wave ring 54 are each of a continuous waveform structure.

It will be understood that the wire diameter, wave height, number of waves, and/or wave angle of the second wave ring 51, the third wave ring 52, and the fourth wave ring 54 may be set according to actual requirements, and are not limited herein.

In some implementations, the wire diameter of the third wave ring 52 is less than the wire diameter of the second wave ring 51. The function of the third wave ring 52 is to supplement the radial support of the second wave ring 51, but the radial support strength of the third wave ring 52 should not be too high, otherwise it may easily cause the first region 100a to irritate the blood vessel or even rupture the blood vessel.

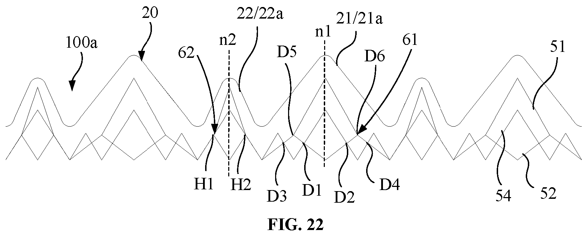

Referring to FIG. 22, in some embodiments, in the first region 100a, there is at least one first intersection point 61 in the region that is coaxial with the first waveform section 21. The first intersection point 61 is formed by the intersection between the first waveform section 21 and the balancing structure 50 or by the balancing structure 50 itself, which can increase the radial support strength of the first region 100a and enable more uniform deployment of the first region 100a in the axial direction. Referring to FIG. 22, in some implementations, the first intersection point 61 is formed by the intersection of at least two of the second wave ring 51, the third wave ring 52, and the fourth wave ring 54 of the balancing structure 50. For example, there are six first intersection points 61, denoted as an intersection point D1, an intersection point D2, an intersection point D3, an intersection point D4, an intersection point D5, and an intersection point D6, respectively. The third wave ring 52 and the fourth wave ring 54 intersect in the region that is coaxial with the first waveform section 21, to form the intersection point D1 and the intersection point D2. The second wave ring 51, the third wave ring 52, and the fourth wave ring 54 intersect in the region that is coaxial with the first waveform section 21, to form the intersection point D3 and the intersection point D4. The second wave ring 51 and the third wave ring 52 intersect in the region that is coaxial with the first waveform section 21, to form the intersection point D5 and the intersection point D6. Thus, the combination of the third wave ring 52 and the fourth wave ring 54, the combination of the second wave ring 51, the third wave ring 52, and the fourth wave ring 54, and the combination of the second wave ring 51 and the third wave ring 52 can each act as an integrated unit to resist radial compressive forces, thereby further improving radial support force, improving the wall apposition of the first region 100a and the proximal end of the covered stent 100, and preventing endoleaks.

In some implementations, the intersection point D1 and the intersection point D2 are symmetrically provided with respect to a first preset straight line, the intersection point D3 and the intersection point D4 are symmetrically provided with respect to the first preset straight line, and the intersection point D5 and the intersection point D6 are symmetrically provided with respect to the first preset straight line, so that the radial support performance of the first region 100a is more uniform, thereby improving the wall apposition of the first region 100a and the covered stent 100. Illustratively, the first preset straight line passes through the wave peak of the first wave 21a, and passes through a wave peak in the region that is coaxial with the first wave 21a in the second wave ring 51. Illustratively, the first preset straight line is shown as a dashed line n1 in FIG. 22.

Referring to FIG. 22, in some embodiments, in the first region 100a, there are a plurality of second intersection points 62 in the region that is coaxial with the second waveform section 22. The number of first intersection points 61 is greater than the number of second intersection points 62. The second intersection point 62 is formed by the intersection between the second waveform section 22 and the balancing structure 50 or by the balancing structure 50 itself. The number of first intersection points 61 is greater than the number of second intersection points 62, which is beneficial for the balancing structure 50 to better balance the radial support strength of the region in the first region 100a that is coaxial with the first waveform section 21 and the radial support strength of the region in the first region 100a that is coaxial with the second waveform section 22, effectively reducing the difference in deployment speed between the region in the first region 100a that is coaxial with the first waveform section 21 and the region in the first region 100a that is coaxial with the second waveform section 22, thereby enabling more uniform deployment of the first region 100a and the covered stent 100 in the circumferential direction, resulting in more uniform forces exerted by the first region 100a and the covered stent 100 on the inner wall of the blood vessel in the circumferential direction, reducing concentrated stress on the inner wall of the blood vessel, and minimizing irritation or damage to the blood vessel during deployment. In addition, it is beneficial for the end section 11 or the first region 100a to better maintain the shape after compression and deployment of the covered stent 100, thereby ensuring good wall apposition of the first region 100a and the covered stent 100.

Referring to FIG. 22, in some implementations, the second intersection point 62 is formed by the intersection of at least two of the second wave ring 51, the third wave ring 52, and the fourth wave ring 54 of the balancing structure 50. For example, there are two second intersection points 62, denoted as an intersection point H1 and an intersection point H2, respectively. The second wave ring 51, the third wave ring 52, and the fourth wave ring 54 intersect in the region that is coaxial with the second waveform section 22, to form the intersection point H1 and the intersection point H2. The combination of the second wave ring 51, the third wave ring 52, and the fourth wave ring 54 can act as an integrated unit to resist the radial compressive force, thereby further improving the radial support force, improving the wall apposition of the first region 100a and the proximal end of the covered stent 100, and preventing endoleaks.

In some implementations, the intersection point H1 and the intersection point H2 are symmetrically provided with respect to a second preset straight line, so that the radial support performance of the first region 100a is more uniform, thereby improving the wall apposition of the first region 100a and the covered stent 100. Illustratively, the second preset straight line passes through the wave peak of the second wave 22a, and passes through a wave peak in the region that is coaxial with the second wave 22a in the second wave ring 51. Illustratively, the second preset straight line is shown as a dashed line n2 in FIG. 22.

Illustratively, the covered stent 100 may be prepared by the following method. A metal wire is braided into a desired waveform. The metal wire may be a nickel-titanium alloy wire with a wire diameter of, for example, 0.35 mm. After heat setting, two ends of the metal wire are sleeved with steel sleeves and fixed through mechanical compression, so that the metal wire and the steel sleeves are connected and tightened, thereby forming a metal ring. After the wave-shaped annular structure is fabricated, a membrane is covered on the surface of a plurality of turns of wave-shaped annular structures sequentially arranged. For example, an e-PTFE membrane may be integrally covered on each of the inner and outer surfaces of the multi-turn wave-shaped annular structure, and the multi-turn wave-shaped annular structure is located between two layers of membranes. The inner and outer e-PTFE membranes are bonded together under high temperature and pressure, thereby fixing the multi-turn wave-shaped annular structure between the two layers of membranes. Of course, when the wave-shaped annular structure is formed by integral cutting of a metal pipe, fixed connection through the steel sleeves is not necessary. An e-PTFE membrane may be integrally covered on the inner or outer surface of the multi-turn wave-shaped annular structure.

In the description of the present application, it should be noted that, unless otherwise clearly specified and limited, the terms "install", "connect", "couple", "mechanically couple", and "engage" should be understood in a broad sense. For example, they may refer to a fixed connection, a detachable connection, or an integral connection. The connection may be mechanical or electrical. They may be direct connection or indirect connection through an intermediate medium, and may include internal communication between two elements or interaction between two elements. Mechanical coupling or engagement between two components includes direct coupling and indirect coupling, for example, direct fixed connection, and connection through a transmission mechanism. For a person skilled in the art, the specific meanings of the foregoing terms in the present application may be understood according to specific circumstances.

In the present application, unless otherwise explicitly stated or limited, a first feature being "above" or "below" a second feature may include that the first feature and the second feature are in direct contact, or that the first feature and the second feature are not in direct contact but are in contact through an additional feature between them. Furthermore, the first feature being "above", "over", and "on" the second feature includes that the first feature is directly above or obliquely above the second feature, or merely indicates that the horizontal height of the first feature is higher than that of the second feature. The first feature being "below", "under", and "beneath" the second feature includes that the first feature is directly below or obliquely below the second feature, or merely indicates that the horizontal height of the first feature is lower than that of the second feature.

The disclosure above provides many different implementations or examples for implementing different structures of the present application. To simplify the disclosure of the present application, components and arrangements of specific examples are described above. Of course, they are merely examples and are not intended to limit the present application. Moreover, in the present application, reference numerals and/or reference letters may be repeated in different examples for the purpose of simplicity and clarity, without indicating a relationship between the various implementations and/or arrangements discussed. In addition, the present invention provides various examples of specific processes and materials, but a person skilled in the art may recognize the application of other processes and/or the use of other materials.

In the description of this specification, the description with reference to the terms such as "one implementation", "some implementations", "schematic implementation", "example", "specific example", or "some examples" means that specific method steps, features, structures, materials, or characteristics described in connection with the implementations or examples are included in at least one implementation or example of the present application. In this specification, schematic expressions of the foregoing terms do not necessarily refer to the same implementation or example. Furthermore, the described specific method steps, features, structures, materials, or characteristics may be combined in any one or more implementations or examples in a suitable manner.

The foregoing description is merely specific implementations of the present application, but the protection scope of the present application is not limited thereto. Various equivalent modifications or substitutions which can be readily thought of by any person skilled in the art within the technical scope disclosed in the present application fall within the protection scope of the present application. Therefore, the protection scope of the present application should be based on the protection scope limited by the claims.

## Claims

1. A covered stent, comprising:
a covered body comprising an end section having an open end;
a first wave ring connected to the end section and comprising a first waveform section and a second waveform section, the radial support strength of the first waveform section being less than the radial support strength of the second waveform section; a region where the end section of the covered stent is located being a first region; and
a balancing structure provided in the first region and configured to increase the radial support strength of a region in the first region that is coaxial with the first waveform section.

2. The covered stent according to claim 1, wherein the balancing structure comprises a second wave ring connected to the end section.

3. The covered stent according to claim 2, wherein an axial region of the second wave ring at least partially overlaps an axial region of the first wave ring.

4. The covered stent according to claim 2, wherein the second wave ring comprises a third wave and a fourth wave; the radial support strength of the third wave is less than the radial support strength of the fourth wave; the fourth wave is axially aligned with the first waveform section, and the third wave is axially aligned with the second waveform section.

5. The covered stent according to claim 4, wherein the first waveform section comprises a first wave, and the second waveform section comprises a second wave; the wave height of the first wave is greater than the wave height of the second wave, and the wave angle of the first wave is equal to the wave angle of the second wave; the wave height of the third wave is equal to the wave height of the fourth wave, and the wave angle of the third wave is greater than the wave angle of the fourth wave.

6. The covered stent according to claim 2, wherein the second wave ring is configured to form a closed structure, and the closed structure is axially aligned with the first waveform section.

7. The covered stent according to claim 6, wherein the balancing structure further comprises a third wave ring connected to the end section, and the third wave ring intersects and cooperates with the second wave ring to form the closed structure.

8. The covered stent according to claim 7, wherein the wire diameter of the third wave ring is less than the wire diameter of the second wave ring.

9. The covered stent according to claim 1, wherein in the first region, there is at least one first intersection point in the region that is coaxial with the first waveform section, wherein the first intersection point is formed by intersection between the first waveform section and the balancing structure or by the balancing structure itself.

10. The covered stent according to claim 9, wherein in the first region, there are a plurality of second intersection points in the region that are coaxial with the second waveform section, and the number of first intersection points is greater than the number of second intersection points, wherein the second intersection point is formed by intersection between the second waveform section and the balancing structure or by the balancing structure itself.

11. The covered stent according to any one of claims 1 to 10, wherein the balancing structure is at least partially provided on an end face of the covered body or adjacent to the end face of the covered body for supporting the end face of the covered body.

12. A delivery system, comprising:
a delivery device; and
the covered stent according to any one of claims 1-11, the delivery device being configured to deliver the covered stent.
